# EUROPEAN PATENT APPLICATION

(11) **EP 4 851 141 A1**
(43) Date of publication of application: **09.12.2026**
(21) Application number: 24921003.0
(22) Date of filing: 02.02.2024
(51) Int. Cl.: C07D 265/36, C07D 471/04, C07C 25/13

(54) **PREPARATION METHOD FOR AND INTERMEDIATE OF 7-FLUOROBENZOXAZINONE COMPOUND**

(71) Applicant: Max (Rudong) Chemicals Co., Ltd., Nantong, Jiangsu 226407 (CN)
(72) Inventor: CHEN, Bangchi, Nantong, Jiangsu 226407 (CN); SHENG, Qiuju, Nantong, Jiangsu 226407 (CN); GUAN, Baochuan, Nantong, Jiangsu 226407 (CN); SUN, Yinwei, Nantong, Jiangsu 226407 (CN); ZHANG, Tianhao, Nantong, Jiangsu 226407 (CN); WANG, Zhongyuan, Nantong, Jiangsu 226407 (CN); ZHANG, Hongwei, Nantong, Jiangsu 226407 (CN)
(74) Representative: Haseltine Lake Kempner LLP
(86) International application number: PCT/CN2024/075437
(87) International publication number: WO 2025/160942

(57) **Abstract**

A method for preparing a 7-fluorobenzoxazinone compound (I) is provided, in which (1) a compound (II) is reacted with a compound (III) in the presence of a Base 1 to form a compound (IV); (2) the compound (IV) is reacted with a compound (V) to form a compound (VI); (3) the compound (VI) is subjected to rearrangement to obtain a compound (VII); and (4) the compound (VII) is subjected to cyclization in the presence of a Base 2 to obtain the 7-fluorobenzoxazinone compound (I), as shown in the following reaction scheme: 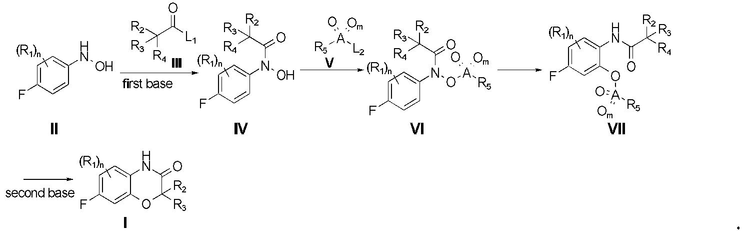 Some intermediates of the 7-fluorobenzoxazinone compound (I) are also provided.

## Description

### CROSS-REFERENCE TO RELATED APPLICATIONS

This application is a continuation of International Patent Application No. PCT/CN2024/075437, filed on February 2, 2024. The content of the aforementioned application, including any intervening amendments thereto, is incorporated herein by reference in its entirety.

### TECHNICAL FIELD

This application relates to organic synthesis, and more particularly to a method for preparing a 7-fluorobenzoxazinone compound and an intermediate thereof.

### BACKGROUND

7-Fluorobenzoxazinone compounds are a class of important chemical intermediates in the preparation of pharmaceuticals and agrochemicals. For example, 7-fluorobenzoxazinone compounds have been used in the preparation of AZD 9977, a drug candidate associated with treatment of heart failure and chronic kidney disease (CN106536491B); in the preparation of antiparasitic drugs (CN105164124B); and in the preparation of STK4 inhibitors for the treatment of hematological malignancies (US10710978B2). In addition, 7-fluorobenzoxazinone compounds can also be used in the preparation of highly-effective herbicides, such as flumioxazin (US4792605A), thidiazimin (DE4008691A1), and trifludimoxazin (CN102459205B).

The 7-fluorobenzoxazinone compounds are mainly prepared through the following three preparation strategies.

In the first strategy, 2-nitro-5-fluorophenol, as a starting material, is subjected to condensation with a haloacetic acid or an ester thereof to form an intermediate, which further undergoes reduction and cyclization to obtain a corresponding 7-fluorobenzoxazinone compound, as shown in the following reaction scheme: 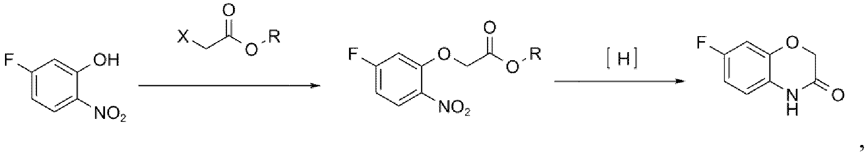 where X represents Cl, Br, etc., and R represents CH₃, C₂H₅, etc.

(Chinese patent application publication No. 108727367A) CN108727367A discloses another preparation method, in which 2-nitro-5-fluorophenol is subjected to a condensation reaction with ethyl bromoacetate in the presence of a base to produce ethyl 2-nitro-5-fluorophenoxyacetate as an intermediate, and the intermediate is then subjected to reduction in the presence of a metal reduction system or a palladium on carbon (Pd/C) catalyst in a hydrogen atmosphere to obtain 7-fluorobenzoxazinone. Such method involves the use of a metal reducing agent or an expensive Pd/C catalyst, resulting in high cost. Moreover, the preparation process is accompanied by the generation of a large amount of waste.

It has also been reported that 2-nitro-5-fluorophenol undergoes condensation with the chloroacetic acid resin in the presence of a base, and the resultant intermediate is subjected to reduction with zinc chloride and cleavage/cyclization to yield 7-fluorobenzoxazinone (Mingzhi Huang et al, Monitoring of Solid-phase Synthesis of 7-Fluoro-2H-1,4-benzoxazine-3(4H)-ones via FTIR Spectroscopy, Chemical Journal of Chinese Universities (2006), 27(11), 2117-2119). However, the involved Wang resin is not commercially available, making this preparation not suitable for the industrial production.

In the second strategy, 2-nitro-5-fluorophenol, as a starting material, is subjected to a condensation reaction with a haloacetonitrile to form a corresponding intermediate, which is then subjected to reduction, cyclization, and hydrolysis to obtain the corresponding 7-fluorobenzoxazinone compound. The reaction scheme is shown as follows: 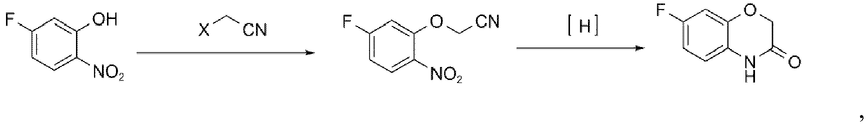 where X represents Cl, Br, etc.

It has also been reported by a literature (Chintakunta Ramesh et al, A simple and facile route for the synthesis of 2H-1,4-benzoxazin-3-(4H)-ones via reductive cyclization of 2-(2-nitrophenoxy)acetonitrile adducts in the presence of Fe/acetic acid, Tetrahedron (2011), 67(6), 1187-1192) that 2-nitro-5-fluorophenol is reacted with chloroacetonitrile or bromoacetonitrile in the presence of a base to yield 2-nitro-5-fluorophenoxyacetonitrile, and then 2-nitro-5-fluorophenoxyacetonitrile is reduced by iron powder to obtain 7-fluorobenzoxazinone. This method struggles with poor economic efficiency due to the use of expensive material chloroacetonitrile or bromoacetonitrile. In addition, the iron powder-triggering reduction may lead to serious pollution due to the generation of a large amount of iron sludge and three wastes.

In the third strategy, 2-nitro-5-fluorophenol is reduced into 2-amino-5-fluorophenol, which is then subjected to condensation with a haloacetyl chloride and cyclization to form the corresponding 7-fluorobenzoxazinone compound. The reaction scheme is shown as follows: 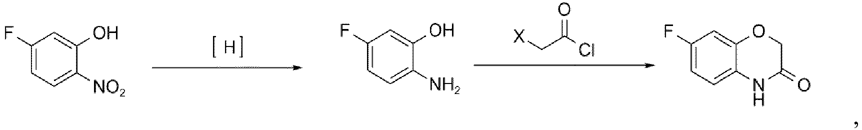 where X represents Cl, Br, etc.

It has also been reported by a literature (Cerda-Cavieres, C.et al, Synthesis, Docking, 3-D-Qsar, and Biological Assays of Novel Indole Derivatives Targeting Serotonin Transporter, Dopamine D2 Receptor, and Mao-A Enzyme: In the Pursuit for Potential Multitarget Directed Ligands, Molecules (2020), 25(20), 4614) that 2-nitro-5-fluorophenol is reduced by iron powder to obtain an intermediate 2-amino-5-fluorophenol, and the intermediate 2-amino-5-fluorophenol is then reacted with chloroacetyl chloride in the presence of a base to undergo cyclization, thereby obtaining the corresponding 7-fluorobenzoxazinone compound. The starting material 2-nitro-5-fluorophenol used in this method is prepared by hydrolysis of 2,4-difluoronitrobenzene (as disclosed in Chinese patent No.106045861B). This hydrolysis reaction has poor chemoselectivity, accompanied by the generation of a large amount of 3-fluoro-4-nitrophenol as a by-product. Such nitrophenol by-products pose a high explosion risk in the separation and purification stage. The hydrolysis reaction also involves the formation of a large amount of corrosive fluoride ions, resulting in high corrosion risk to the involved reaction apparatuses. In addition, the iron powder reducing agent used in this method results in generation of a large amount of iron sludge, which will complicate the post-treatment operation and cause serious pollution, thereby hindering the industrial application.

### SUMMARY

To this regard, an object of the disclosure is to provide a method for preparing a 7-fluorobenzoxazinone compound and an intermediate thereof. The method has readily-available raw materials, simple operation, less generation of waste, and is thus suitable for industrial production.

Technical solutions of the present disclosure are described as follows.

In a first aspect, this application provides a method for preparing a 7-fluorobenzoxazinone compound I, including:
step (1) reacting a compound II with a compound III in the presence of a Base 1 to form a compound IV;
step (2) reacting the compound IV with a compound V to form a compound VI;
step (3) subjecting the compound VI to rearrangement to obtain a compound VII; and
step (4) subjecting the compound VII to cyclization in the presence of a Base 2 to obtain the 7-fluorobenzoxazinone compound I, as shown in the following reaction scheme:

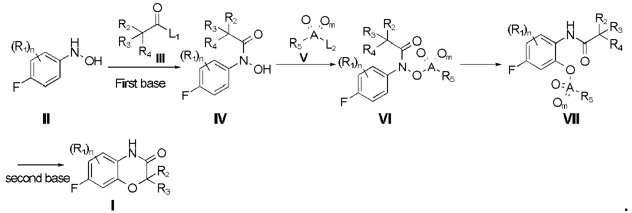

In the above following reaction scheme, R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino. In some embodiments, R₁ is hydrogen, halogen, unsubstituted or substituted C₁-C₃ alkyl, unsubstituted or substituted C₆-C₁₂ aryl, unsubstituted or substituted heteroaryl, cyano, nitro, or substituted amino. In some embodiments, R₁ is selected from the group consisting of hydrogen, nitro, and substituted amino.

R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy. In some embodiments, R₂ and R₃ are each independently hydrogen, halogen, unsubstituted or substituted C₁-C₃ alkyl, or unsubstituted or substituted C₁-C₃ alkoxy. In some embodiments, R₂ and R₃ are each independently selected from the group consisting of hydrogen, F, Cl, and Br.

R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy. In some embodiments, R₄ is selected from the group consisting of hydrogen, halogen, unsubstituted or substituted C₁-C₃ alkylsulfonyloxy, or unsubstituted or substituted C₆-C₁₂ arylsulfonyloxy. In some embodiments, R₄ is selected from the group consisting of hydrogen, Cl, Br, methanesulfonyloxy, and benzenesulfonyloxy.

A is C or S.
when A is C, m is 0.
when A is S, m is 0 or 1.

R₅ is hydrogen, unsubstituted or substituted C₁-C₈ alkyl, unsubstituted or substituted C₃-C₁₀ cycloalkyl, unsubstituted or substituted C1-C8 alkoxy, unsubstituted or substituted C₆-C₁₂ aryl, or unsubstituted or substituted heteroaryl. In some embodiments, R₅ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl. In some embodiments, R₅ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, unsubstituted phenyl and substituted phenyl.

The unsubstituted or substituted heteroaryl group is a heteroaryl group containing 1 to 4 heteroatoms selected from the group consisting of nitrogen, oxygen, and sulfur atoms, such as pyrazolyl, thiazolyl, thienyl, pyridyl, and pyrimidinyl.

L₁ and L₂ are each independently a leaving group. In some embodiments, the leaving group is R₆C(=O)O- or halogen. In some embodiments, the leaving group is Cl, CH₃C(=O)O-, CCl₃C(=O)O-, or ClCH₂C(=O)O-.

In some embodiments, R₆ is hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl. In some embodiments, R₆ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl. In some embodiments, R₆ is methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, or unsubstituted or substituted phenyl.

In some embodiments, n is 0, 1, 2, 3, or 4; and when n is greater than 1, a plurality of R₁ are the same or different.

In some embodiments, the step (2) is optionally performed in the presence of a Base 3.

In some embodiments, the Base 1, the Base 2 and the Base 3 are each independently selected from the group consisting of an inorganic base, an organic base and a combination thereof. The inorganic base is selected from the group consisting of an alkali metal carbonate, an alkali metal bicarbonate, an alkali metal phosphate, an alkali metal hydroxide, an alkali metal hydride, and an alkali metal amide, an alkaline-earth metal carbonate, an alkaline-earth metal bicarbonate, an alkaline-earth metal phosphate, an alkaline-earth metal hydroxide, an alkaline-earth metal oxide, an alkaline-earth metal hydride, and an alkaline-earth metal amide. In some embodiments, the inorganic base is selected from the group consisting of the alkali metal carbonate, the alkali metal bicarbonate, the alkali metal hydroxide, the alkaline-earth metal carbonate, the alkaline-earth metal bicarbonate, and the alkaline-earth metal hydroxide. In some embodiments, the inorganic base is selected from the group consisting of sodium carbonate, sodium bicarbonate, potassium carbonate, potassium bicarbonate, sodium hydroxide, and potassium hydroxide.

In some embodiments, the organic base is an organic salt or an organic amine. The organic salt is selected from the group consisting of an alkyl carboxylate, an aryl carboxylate, an alkoxide salt, a metal alkyl compound and a metal aryl compound. The organic amine is an alkylamine or an arylamine. The alkylamine is a tertiary amine. In some embodiments, the alkylamine is triethylamine. The arylamine is a pyridylamine compound. In some embodiments, the arylamine is pyridine or methylpyridine.

In some embodiments, the compound III in the step (1) is an acyl halide or an acid anhydride. The acyl halide is unsubstituted or substituted C₁-C₈ alkyl acyl halide, unsubstituted or substituted C₃-C₁₀ cycloalkyl acyl halide, unsubstituted or substituted C₆-C₁₂ aryl acyl halide, or unsubstituted or substituted heteroaryl acyl halide. In some embodiments, the compound III is selected from the group consisting of acetyl chloride, chloroacetyl chloride, bromoacetyl chloride, dichloroacetyl chloride, trichloroacetyl chloride, tribromoacetyl chloride, acetic anhydride, chloroacetic anhydride, dichloroacetic anhydride, trichloroacetic anhydride, bromoacetic anhydride, 2-chloro-2,2-difluoroacetyl chloride, 2-bromo-2,2-difluoroacetyl chloride, and 2-bromo-2,2-dichloroacetyl chloride.

In some embodiments, the solvent used in the step (1) is an organic solvent or a mixture of the organic solvent and water. The organic solvent is selected from the group consisting of an aromatic hydrocarbon solvent, an amide solvent, a sulfoxide or sulfone solvent, a halogenated hydrocarbon solvent, an ether solvent, an ester solvent and a combination thereof. The aromatic hydrocarbon solvent includes, for example, a C₆-C₁₂ aromatic hydrocarbon solvent, such as toluene, xylene, and trimethylbenzene. The amide solvent includes, for example, a C₁-C₆ amide solvent, such as dimethylformamide and dimethylacetamide. The sulfoxide or sulfone solvent includes, for example, a C₁-C₆ sulfoxide or sulfone solvent, such as dimethyl sulfoxide and sulfolane. The halogenated hydrocarbon solvent includes, for example, dichloromethane, chloroform, carbon tetrachloride, and dichloroethane. The ether solvent includes, for example, methyl tert-butyl ether, tetrahydrofuran, and methyltetrahydrofuran. The ester solvent includes, for example, ethyl acetate. In some embodiments, the solvent used in step (1) is selected from the group consisting of toluene, tetrahydrofuran, methyltetrahydrofuran, and ethyl acetate.

In some embodiments, the compound V in the step (2) is an acyl halide or an acid anhydride. The acyl halide is unsubstituted or substituted C₁-C₈ alkyl acyl halide, unsubstituted or substituted C₃-C₁₀ cycloalkyl acyl halide, unsubstituted or substituted C₆-C₁₂ aryl acyl halide, or unsubstituted or substituted heteroaryl acyl halide. In some embodiments, the compound V is selected from the group consisting of acetyl chloride, chloroacetyl chloride, bromoacetyl chloride, dichloroacetyl chloride, trichloroacetyl chloride, tribromoacetyl chloride, cyclopropanecarbonyl chloride, acetic anhydride, chloroacetic anhydride, dichloroacetic anhydride, trichloroacetic anhydride, bromoacetic anhydride, 2-chloro-2,2-difluoroacetyl chloride, 2-bromo-2,2-difluoroacetyl chloride, 2-bromo-2,2-dichloroacetyl chloride, benzoyl chloride, thiophene acyl chloride methanesulfonyl chloride, and benzenesulfonyl chloride.

In some embodiments, the step (2) is optionally performed in the presence of a solvent, which is selected from the group consisting of an aromatic hydrocarbon solvent, an amide solvent, a sulfoxide or sulfone solvent, a halogenated hydrocarbon solvent, an ether solvent, an ester solvent and a combination thereof. The aromatic hydrocarbon solvent includes, for example, a C₆-C₁₂ aromatic hydrocarbon solvent, such as toluene, xylene, and trimethylbenzene. The amide solvent includes, for example, a C₁-C₆ amide solvent, such as dimethylformamide and dimethylacetamide. The sulfoxide or sulfone solvent includes, for example, a C₁-C₆ sulfoxide or sulfone solvent, such as dimethyl sulfoxide and sulfolane. The halogenated hydrocarbon solvent includes, for example, dichloromethane, chloroform, carbon tetrachloride, and dichloroethane. The ether solvent includes, for example, methyl tert-butyl ether, tetrahydrofuran, and methyltetrahydrofuran. The ester solvent includes, for example, ethyl acetate. In some embodiments, the solvent used in step (2) is selected from the group consisting of toluene, tetrahydrofuran, methyltetrahydrofuran, and ethyl acetate.

In some embodiments, the solvent used in the step (3) and step (4) may be selected from the group consisting of an aromatic hydrocarbon solvent, an amide solvent, a sulfoxide or sulfone solvent, a halogenated hydrocarbon solvent, an ether solvent, an ester solvent and a combination thereof. The aromatic hydrocarbon solvent includes, for example, a C₆-C₁₂ aromatic hydrocarbon solvent, such as toluene, xylene, and trimethylbenzene. The amide solvent includes, for example, a C₁-C₆ amide solvent, such as dimethylformamide and dimethylacetamide. The sulfoxide or sulfone solvent includes, for example, a C₁-C₆ sulfoxide or sulfone solvent, such as dimethyl sulfoxide and sulfolane. The halogenated hydrocarbon solvent includes, for example, dichloromethane, chloroform, carbon tetrachloride, and dichloroethane. The ether solvent includes, for example, methyl tert-butyl ether, tetrahydrofuran, and methyltetrahydrofuran. The ester solvent includes, for example, ethyl acetate. In some embodiments, the solvent used in the step (3) and step (4) is selected from the group consisting of toluene, tetrahydrofuran, methyltetrahydrofuran, and ethyl acetate.

In some embodiments, the 7-fluorobenzoxazinone compound I is prepared from the compound II in the presence of the Base 1 in a one-pot manner.

In some embodiments, the 7-fluorobenzoxazinone compound I is prepared from the compound IV or the compound VI in a one-pot manner.

In the presence of the Base 2, the compound VII is prepared by reacting the compound IV with the compound VI in a one-pot manner.

In a second aspect, this application provides an intermediate compound for preparing the 7-fluorobenzoxazinone compound I, represented by formula (IV): 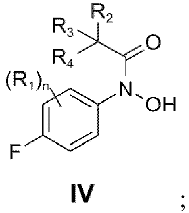
wherein R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino; preferably, R₁ is hydrogen, halogen, unsubstituted or substituted C₁-C₃ alkyl, unsubstituted or substituted C₆-C₁₂ aryl, unsubstituted or substituted heteroaryl, cyano, nitro, or substituted amino; more preferably, R₁ is selected from the group consisting of hydrogen, nitro and substituted amino;
R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy; preferably, R₂ and R₃ are each independently hydrogen, halogen, unsubstituted or substituted C₁-C₃ alkyl, or unsubstituted or substituted C₁-C₃ alkoxy; more preferably, R₂ and R₃ are each independently selected from the group consisting of hydrogen, F, Cl, and Br; and
R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy; preferably, R₄ is halogen, unsubstituted or substituted C₁-C₃ alkylsulfonyloxy, or unsubstituted or substituted C₆-C₁₂ arylsulfonyloxy; more preferably, R₄ is selected from the group consisting of Cl, Br, methanesulfonyloxy, and benzenesulfonyloxy.

In a third aspect, this application provides an intermediate compound for preparing the 7-fluorobenzoxazinone compound I, represented by formula (VI): 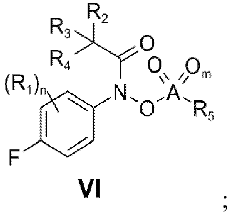
wherein R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino;
R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy;
R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy;
R₅ is hydrogen, unsubstituted or substituted C₁-C₈ alkyl, unsubstituted or substituted C₃-C₁₀ cycloalkyl, unsubstituted or substituted C1-C8 alkoxy, unsubstituted or substituted C₆-C₁₂ aryl, or unsubstituted or substituted heteroaryl;
A is C or S;
when A is C, m is 0;
when A is S, m is 0 or 1; and
n is 0, 1, 2, 3, or 4; and when n is greater than 1, a plurality of R₁ are the same or different.

In some embodiments, when R₁ is hydrogen,
R₂ and R₃ are each independently hydrogen or halogen; preferably, R₂ and R₃ are each independently selected from the group consisting of hydrogen, F, Cl, and Br;
R₄ is hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy; preferably, R₄ is halogen, unsubstituted or substituted C₁-C₃ alkylsulfonyloxy, or unsubstituted or substituted C₆-C₁₂ arylsulfonyloxy; more preferably, R₄ is selected from the group consisting of Cl, Br, methanesulfonyloxy, and benzenesulfonyloxy; and
R₅ is unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl; preferably, R₅ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl; more preferably, R₅ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, phenyl, and substituted phenyl.

In some embodiments, the unsubstituted or substituted heteroaryl group is a heteroaryl group containing 1 to 4 heteroatoms selected from the group consisting of nitrogen, oxygen, and sulfur atoms, such as pyrazolyl, thiazolyl, thienyl, pyridyl, and pyrimidinyl.

In a fourth aspect, this application provides an intermediate compound for preparing a 7-fluorobenzoxazinone compound I, represented by formula (VII): 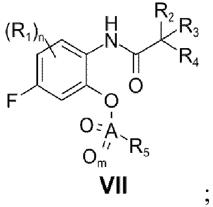
wherein R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino;
R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy;
R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy;
R₅ is hydrogen, unsubstituted or substituted C₁-C₈ alkyl, unsubstituted or substituted C₃-C₁₀ cycloalkyl, unsubstituted or substituted C1-C8 alkoxy, unsubstituted or substituted C₆-C₁₂ aryl, or unsubstituted or substituted heteroaryl;
A is C or S;
when A is C, m is 0;
when A is S, m is 0 or 1; and
n is 0, 1, 2, 3, or 4; and when n is greater than 1, a plurality of R₁ are the same or different.

In some embodiments, when R₁ is hydrogen,
R₂ and R₃ are each independently hydrogen or halogen; preferably, R₂ and R₃ are each independently selected from the group consisting of hydrogen, F, Cl and Br;
R₄ is hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy; preferably, R₄ is halogen, unsubstituted or substituted C₁-C₃ alkylsulfonyloxy, or unsubstituted or substituted C₆-C₁₂ arylsulfonyloxy; more preferably, R₄ is selected from the group consisting of Cl, Br, methanesulfonyloxy, and benzenesulfonyloxy; and
R₅ is unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl; preferably, R₅ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl; more preferably, R₅ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, phenyl, and substituted phenyl.

In some embodiments, the unsubstituted or substituted heteroaryl group is a heteroaryl group containing 1 to 4 heteroatoms selected from the group consisting of nitrogen, oxygen, and sulfur atoms, such as pyrazolyl, thiazolyl, thienyl, pyridyl, and pyrimidinyl.

Compared to the prior art, the present disclosure has the following beneficial effects.
(1) The disclosure provides a novel strategy for preparing the 7-fluorobenzoxazinone compound and novel intermediate compounds.
(2) The starting materials are inexpensive and readily available, and no special reagents, such as expensive catalysts, or special reaction equipment are required.
(3) The reaction conditions are mild and the operation is convenient.
(4) The process is safe and generates less three wastes
(5) The process is suitable for industrial production.

The groups are defined as follows.

As used herein, the term "halogen" refers to fluorine, chlorine, bromine, or iodine.

As used herein, the term "substituted" in the expression "unsubstituted or substituted" means that one or more hydrogen atoms attached to a carbon atom or a nitrogen atom are each independently replaced by a substituent selected from the group consisting of halogen, C₁-C₆ alkyl, C₁-C₆ haloalkyl, C₃-C₉ cycloalkyl, C₃-C₉ halocycloalkyl, C₂-C₆ alkenyl, C₂-C₆ haloalkenyl, C₂-C₆ alkynyl, C₂-C₆ haloalkynyl, C₁-C₆ alkoxy, C₁-C₆ haloalkoxy, C₁-C₆ alkylthio, C₁-C₆ haloalkylthio, C₁-C₆ alkylsulfinyl, C₁-C₆ haloalkylsulfinyl, C₁-C₆ alkylsulfonyl, C₁-C₆ haloalkylsulfonyl, C₂-C₇ alkylcarbonyl, C₂-C₇ haloalkylcarbonyl, C₂-C₇ alkylcarbonyloxy, C₂-C₇ haloalkylcarbonyloxy, C₁-C₆ alkylsulfonyloxy, C₁-C₆ haloalkylsulfonyloxy, C₂-C₇ alkoxycarbonyl, C₂-C₇ haloalkoxycarbonyl, C₂-C₇ alkylcarbonylamino, C₂-C₇ haloalkylcarbonylamino, C₂-C₇ alkoxycarbonylamino, C₂-C₇ haloalkoxycarbonylamino, C₂-C₇ alkylaminocarbonyl, C₂-C₇ haloalkylaminocarbonyl, C₁-C₆ alkylamino, C₁-C₆ haloalkylamino, C₂-C₆ alkenylamino, C₂-C₆ haloalkenylamino, C₂-C₆ alkynylamino, C₂-C₆ haloalkynylamino, C₃-C₉ cycloalkylamino, C₃-C₉ halocycloalkylamino, C₃-C₇ alkenylaminocarbonyl, C₃-C₇ haloalkenylaminocarbonyl, C₃-C₇ alkynylaminocarbonyl, C₃-C₇ haloalkynylaminocarbonyl, C₄-C₁₀ cycloalkylaminocarbonyl, C₄-C₁₀ halocycloalkylaminocarbonyl, amino, carbamoyl, sulfamoyl, cyano, nitro, hydroxy, carboxy, optionally substituted phenyl, optionally substituted heterocyclyl, optionally substituted benzyl, optionally substituted phenylcarbonyl, and optionally substituted phenylamino.

### DETAILED DESCRIPTION OF EMBODIMENTS

### EXAMPLE 1

18.45 g of N-(4-fluorophenyl) hydroxylamine was dissolved in 40 mL of tetrahydrofuran. The mixture was cooled to a temperature from -15°C to -20°C. 19 g of triethylamine was added followed by 17 g of chloroacetyl chloride. The mixture was reacted at about -15°C until the reaction was completed as determined by the in-process monitoring. After usual workup, 29.5 g of 2-chloro-N-(4-fluorophenyl)-N-hydroxyacetamide (97% yield) was obtained.

¹H NMR (CDCl₃, 500 MHz, TMS) δ 10.73 (s, 1H), 6.90-6.87 (m, 2H), 6.82-6.78 (m, 2H), 4.59 (s, 2H).

### EXAMPLE 2

17 g of N-(4-fluorophenyl) hydroxylamine was dissolved in 40 mL of ethyl acetate. The mixture was cooled to -5°C-0°C. 16.8 g of chloroacetyl chloride was added dropwise followed by 145.6 g of a 10 wt.% aqueous sodium bicarbonate solution. The mixture was reacted at the same temperature ranging from -5°C-0°C until the reaction was completed. After usual workup, 26.2 g of 2-chloro-N-(4-fluorophenyl)-N-hydroxyacetamide (98% yield) was obtained.

### EXAMPLE 3

12 g of N-(4-fluorophenyl) hydroxylamine was dissolved in 30 mL of 2-methyltetrahydrofuran. The mixture was cooled to a temperature from -15°C to -20°C. 10.5 g of triethylamine was added followed by 7.9 g of acetyl chloride. The mixture was reacted at about -15°C until the reaction was completed as determined by the in-process monitoring. After usual workup, 16.2 g of N-(4-fluorophenyl)-N-hydroxyacetamide (96% yield) was obtained.

### EXAMPLE 4

17 g of N-(4-fluorophenyl) hydroxylamine was dissolved in 40 mL of toluene.The mixture was cooled to a temperature from -20°C to -15°C.13.6 g of acetyl chloride was added dropwise followed by 17.04 g of triethylamine. The mixture was reacted at -20°C to -15°C until the reaction was completed. After usual workup, 22 g of N-(4-fluorophenyl)-N-hydroxyacetamide (97% yield) was obtained.

¹H NMR (CDCl₃, 500 MHz, TMS) δ 10.47 (s, 1H), 7.68-7.60 (m, 2H), 7.25-7.16 (m, 2H), 2.18 (s, 3H).

### EXAMPLE 5

5 g of 2-chloro-N-(4-fluorophenyl)-N-hydroxyacetamide prepared in Example 1 or 2 was added to 20 g of acetic anhydride.The mixture was reacted in an oil bath at 55°C until the reaction was completed as determined by the in-process monitoring. After usual workup, 6 g of N-acetoxy-2-chloro-N-(4-fluorophenyl) acetamide (99% yield) was obtained.

¹H NMR (CDCl₃, 500 MHz, TMS) δ 7.60 (s, 2H), 7.34 (s, 2H), 4.49 (br, 2H), 2.26 (s, 3H).

### EXAMPLE 6

4 g of 2-chloro-N-(4-fluorophenyl)-N-hydroxyacetamide prepared in Example 1 or 2 was dissolved in 20 mL of chloroform, to which 3.2 g of ethyl chloroformate and 2.5 g of pyridine were added dropwise at room temperature. After the dropwise addition, the reaction mixture was reacted at room temperature until completion of the reaction as determined by HPLC monitoring. After usual workup, 5.4 g of 2-chloro-N-((ethoxycarbonyl)oxy)-N-(4-fluorophenyl) acetamide (99% yield) was obtained.

¹H NMR (CDCl₃, 500 MHz, TMS) δ 7.62 (s, 2H), 7.39-7.36 (m, 2H), 4.44 (br, 2H), 4.31 (q, *J* = 7.0 Hz, 2H), 1.27 (t, *J* = 7.0 Hz, 3H).

### EXAMPLE 7

4 g of 2-chloro-N-(4-fluorophenyl)-N-hydroxyacetamide prepared in Example 1 or 2 was dissolved in 20 mL of chloroform, to which 3.2 g of 4-nitrobenzoyl chloride and 2.5 g of pyridine were added dropwise at room temperature. After the dropwise addition, the reaction mixture was reacted at room temperature until completion of the reaction as determined by HPLC monitoring. After usual workup, 5.4 g of 2-chloro-N-(4-fluorophenyl)-N-(4-nitrobenzoyloxy) acetamide (99% yield) was obtained.

¹H NMR (CDCl₃, 500 MHz, TMS) δ 8.39-8.41(d, 2H), 8.28-8.32(d, 2H), 7.72(m, 2H),7.72(m, 2H),7.38(m, 2H), 4.58(s,2H).

### EXAMPLE 8

20.0 g of 2-chloro-N-(4-fluorophenyl)-N-hydroxyacetamide prepared according to Example 1 was dissolved in 40 mL of chloroform, to which 19.6 g of trichloroacetyl chloride was added dropwise at room temperature. After the dropwise addition, the reaction mixture was reacted under stirring until completion of the reaction. After usual workup, 25.6 g of 2-(2-chloroacetamido)-5-fluorophenyl 2,2,2-trichloroacetate (75% yield) was obtained.

¹H NMR (500 MHz, CDCl₃) δ 8.44 (s, 1H), 8.22 (dd, *J* = 9.2, 5.7 Hz, 1H), 7.18 (dd, *J* = 8.4, 2.8 Hz, 1H), 7.09 (ddd, *J* = 9.3, 7.9, 2.8 Hz, 1H), 4.21 (s, 2H).

### EXAMPLE 9

### 2-(2-Chloroacetamido)-5-fluorophenyl benzoate

19.3 g of N-benzoyloxy-2-chloro-N-(4-fluorophenyl) acetamide prepared according to Example 5 was dissolved in 40 mL of toluene, and heated to reflux until completion of the reaction. After usual workup, 22.5 g of 2-(2-chloroacetamido)-5-fluorophenyl benzoate (72% yield) was obtained.

¹H NMR (CDCl₃, 500 MHz, TMS) δ 9.97 (s, 1H), 8.16-8.15 (m, 2H), 7.80-7.75 (m, 2H), 7.61 (s, 2H), 7.39-7.37 (m, 1H), 7.20 (m, 1H), 4.24 (s, 2H).

### EXAMPLE 10

### 2-(2-Bromoacetamido)-5-fluorophenyl benzenesulfonate

20.0 g of N-benzenesulfonyloxy-2-bromo-N-(4-fluorophenyl) acetamide prepared according to Example 5 was dissolved in 40 mL of ethyl acetate, and the solution was refluxed under stirring until completion of the reaction. After usual workup,23.9 g of 2-(2-bromoacetamido)-5-fluorophenyl benzenesulfonate (71% yield) was obtained.

¹H NMR (CDCl₃, 500 MHz, TMS) δ 9.88 (s, 1H), 7.87-7.85 (m, 2H), 7.82-7.74 (m, 2H), 7.66-7.63 (m, 2H), 7.23-7.20 (m, 2H), 3.91 (s, 2H).

### EXAMPLE 11

### 2-(2-Chloroacetamido)-5-fluorophenyl thiophene-2-carboxylate

15.7 g of N-(thiophene-2-carbonyloxy)-2-chloro-N-(4-fluorophenyl) acetamide prepared according to Example 5 was dissolved in 40 mL of toluene, to which 7.0 g of potassium carbonate was added under stirring. The reaction mixture was refluxed until completion of the reaction. After usual workup,23.9 g of 2-(2-chloroacetamido)-5-fluorophenyl thiophene-2-carboxylate (76% yield) was obtained.

¹H NMR (CDCl₃, 500 MHz, TMS) δ 9.92 (s, 1H), 8.11-8.10 (m, 1H), 8.03-8.02 (m, 1H), 7.76-7.73 (m ,1H), 7.40-7.38 (m, 1H), 7.32-7.31 (m, 1H), 7.22-7.18 (m, 1H), 4.23 (s, 2H).

### EXAMPLE 12

According to the procedure of Example 9, 2-(2-bromo-2,2-difluoroacetamido)-5-fluorophenyl acetate was prepared (67% yield).

¹H NMR (500 MHz, DMSO) δ 9.81 (s, 1H), 7.70 (dd, *J* = 8.7, 6.1 Hz, 1H), 7.18 (dd, *J* = 9.3, 2.6 Hz, 1H), 7.13 (td, *J* = 8.6, 2.7 Hz, 1H), 4.29 (s, 2H), 1.94 - 1.86 (m, 1H), 1.08 (d, *J* = 6.4 Hz, 2H), 1.06 (s, 2H).

### EXAMPLE 13

According to the procedure of Example 9, 2-(2-chloroacetamido)-5-fluorophenyl cyclopropanecarboxylate was prepared (70% yield).

¹H NMR (500 MHz, DMSO) δ 9.81 (s, 1H), 7.70 (dd, *J* = 8.7, 6.1 Hz, 1H), 7.18 (dd, *J* = 9.3, 2.6 Hz, 1H), 7.13 (td, *J* = 8.6, 2.7 Hz, 1H), 4.29 (s, 2H), 1.94 - 1.86 (m, 1H), 1.08 (s, 2H), 1.06 (s, 2H).

### EXAMPLE 14

4.2 g of 2-(2-chloroacetamido)-5-fluorophenyl 2,2,2-trichloroacetate prepared in Example 8 was dissolved in 40 mL of ethanol, to which 28.8 g of aqueous sodium hydroxide (NaOH) solution was slowly added dropwise under stirring. After the dropwise addition, the reaction mixture was reacted under stirring until completion of the reaction. After usual workup, 14.8 g of 7-fluoro-2H-benzo[b][1,4]oxazin-3(4H)-one (97% yield) was obtained.

### EXAMPLE 15

### One-pot preparation of 7-fluoro-2H-benzo[b][1,4]oxazin-3(4H)-one

26.0 g of 2-chloro-N-(4-fluorophenyl)-N-hydroxyacetamide prepared in Example 1 was dissolved in 40 mL of toluene, heated to 50 °C, and dropwise added with 24.2 g of trichloroacetyl chloride slowly. After the dropwise addition, the reaction mixture was maintained at 50°C for reaction. After the reaction was completed, the reaction mixture was washed with water. To the organic layer was added dropwise 51.4 g of an aqueous NaOH solution. After the addition, the reaction mixture was stirred and reacted until the reaction was complete. After usual workup, 20.1 g of 7-fluoro-2H-benzo[b][1,4]oxazin-3(4H)-one (94% yield) was obtained.

Described above are merely illustrative, and are not intended to limit the scope of the present disclosure. It should be understood that various modifications, changes and replacements made by those skilled in the art without departing from the spirit of the disclosure shall fall within the scope of the present disclosure defined by the appended claims.

## Claims

1. A method for preparing a 7-fluorobenzoxazinone compound I, comprising:
step (1) reacting a compound II with a compound III in the presence of a Base 1 to form a compound IV;
step (2) reacting the compound IV with a compound V to form a compound VI;
step (3) subjecting the compound VI to rearrangement to obtain a compound VII;
and
step (4) subjecting the compound VII to cyclization in the presence of a Base 2 to obtain the 7-fluorobenzoxazinone compound I, as shown in the following reaction scheme: 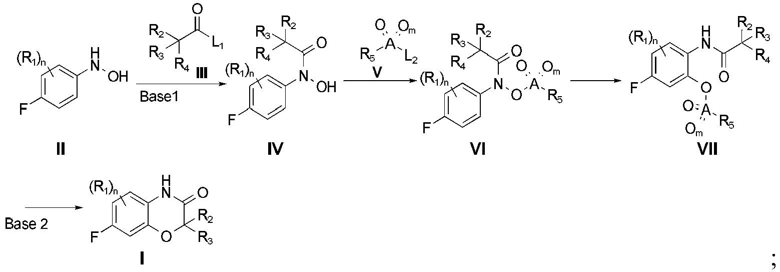
wherein R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino;
R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy;
R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy;
A is C or S;
when A is C, m is 0;
when A is S, m is 0 or 1;
R₅ is hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl;
L₁ and L₂ are each independently a leaving group;
n is 0, 1, 2, 3, or 4; and when n is greater than 1, a plurality of R₁ are the same or different; and
the Base 1 and the Base 2 are each independently selected from the group consisting of an inorganic base, an organic base and a combination thereof.

2. The method according to claim 1, **characterized in that**
R₁ is hydrogen, halogen, unsubstituted or substituted C₁-C₃ alkyl, unsubstituted or substituted C₆-C₁₂ aryl, unsubstituted or substituted heteroaryl, cyano, nitro, or substituted amino;
R₂ and R₃ are each independently hydrogen, halogen, unsubstituted or substituted C₁-C₃ alkyl, or unsubstituted or substituted C₁-C₃ alkoxy;
R₄ is hydrogen, halogen, unsubstituted or substituted C₁-C₃ alkoxy, unsubstituted or substituted C₁-C₃ alkylsulfonyloxy, or unsubstituted or substituted C₆-C₁₂ arylsulfonyloxy;
R₅ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl;
L₁ and L₂ are each independently halogen or R₆C(=O)O-;
R₆ is hydrogen, unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl;
the inorganic base is selected from the group consisting of an alkali metal carbonate, an alkali metal bicarbonate, an alkali metal phosphate, an alkali metal hydroxide, an alkali metal hydride, an alkali metal amide, an alkaline-earth metal carbonate, an alkaline-earth metal bicarbonate, an alkaline-earth metal phosphate, an alkaline-earth metal hydroxide, an alkaline-earth metal oxide, an alkaline-earth metal hydride, and an alkaline-earth metal amide; and
the organic base is an organic salt or an organic amine.

3. The method according to claim 2, **characterized in that** R₁ is selected from the group consisting of hydrogen, nitro and substituted amino;
R₂ and R₃ are each independently selected from the group consisting of hydrogen, F, Cl and Br;
R₄ is selected from the group consisting of hydrogen, Cl, Br, methanesulfonyloxy and benzenesulfonyloxy;
R₅ is methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, unsubstituted or substituted phenyl, unsubstituted or substituted pyrazolyl, unsubstituted or substituted thiazolyl, unsubstituted or substituted thienyl, unsubstituted or substituted pyridyl, or unsubstituted or substituted pyrimidinyl;
R₆ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, unsubstituted phenyl and substituted phenyl;
the inorganic base is selected from the group consisting of the alkali metal carbonate, the alkali metal bicarbonate, the alkali metal hydroxide, the alkaline-earth metal carbonate, the alkaline-earth metal bicarbonate and the alkaline-earth metal hydroxide;
the organic salt is selected from the group consisting of an alkyl carboxylate, an aryl carboxylate, an alkoxide salt, a metal alkyl compound, and a metal aryl compound; and
the organic amine is an alkylamine or an arylamine.

4. The method according to claim 3, **characterized in that** the inorganic base is selected from the group consisting of sodium carbonate, sodium bicarbonate, potassium carbonate, potassium bicarbonate, sodium hydroxide and potassium hydroxide;
the alkylamine is a tertiary amine, preferably triethylamine; and
the arylamine is a pyridylamine compound, preferably pyridine or methylpyridine.

5. The method according to claim 1, **characterized in that** the 7-fluorobenzoxazinone compound I is prepared from the compound II in the presence of the Base 1 in a one-pot manner; or
the 7-fluorobenzoxazinone compound I is prepared from the compound IV or the compound (VI) in a one-pot manner.

6. The method according to claim 1, **characterized in that** the compound VII is prepared from the compound IV and the compound VI in a one-pot manner.

7. A compound, represented by formula (IV): 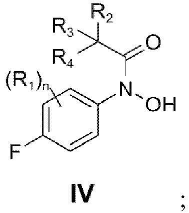
**characterized in that** R₁ is hydrogen, halogen, unsubstituted or substituted C₁-C₈ alkyl, unsubstituted or substituted C₆-C₁₂ aryl, unsubstituted or substituted heteroaryl, unsubstituted or substituted acyl, cyano, substituted hydroxy, substituted mercapto, substituted sulfinyl, substituted sulfonyl, nitro, or substituted amino; preferably, R₁ is hydrogen, halogen, unsubstituted or substituted C₁-C₃ alkyl, unsubstituted or substituted C₆-C₁₂ aryl, unsubstituted or substituted heteroaryl, cyano, nitro, or substituted amino; and more preferably, R₁ is selected from the group consisting of hydrogen, nitro and substituted amino;
R₂ and R₃ are each independently hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted C₆-C₁₂ aryloxy, unsubstituted or substituted heteroaryl, or unsubstituted or substituted heteroaryloxy; preferably, R₂ and R₃ are each independently hydrogen, halogen, unsubstituted or substituted C₁-C₃ alkyl, or unsubstituted or substituted C₁-C₃ alkoxy; and more preferably, R₂ and R₃ are each independently selected from the group consisting of hydrogen, F, Cl and Br; and
R₄ is hydrogen, halogen, unsubstituted or substituted C₁-C₈ alkyl, unsubstituted or substituted C₁-C₈ alkoxy, C₁-C₈ acyloxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy; preferably, R₄ is halogen, unsubstituted or substituted C₁-C₃ alkylsulfonyloxy, or unsubstituted or substituted C₆-C₁₂ arylsulfonyloxy; and more preferably, R₄ is selected from the group consisting of Cl, Br, methanesulfonyloxy and benzenesulfonyloxy.

8. A compound, represented by formula (VI): 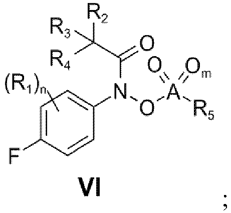
**characterized in that** R₁, R₂, R₃, R₄, R₅, A, m and n are defined as above;
preferably, when R₁ is hydrogen, R₂ and R₃ are each independently hydrogen or halogen;
R₄ is hydrogen, halogen, unsubstituted or substituted C₁-C₈ alkyl, unsubstituted or substituted C₁-C₈ alkoxy, unsubstituted or substituted C₆-C₁₂ aryl, unsubstituted or substituted heteroaryl, unsubstituted or substituted C₁-C₈ alkylsulfonyloxy, or unsubstituted or substituted C₆-C₁₂ arylsulfonyloxy; and
R₅ is unsubstituted or substituted C₁-C₈ alkyl, unsubstituted or substituted C₃-C₁₀ cycloalkyl, unsubstituted or substituted C₁-C₈ alkoxy, unsubstituted or substituted C₆-C₁₂ aryl, or unsubstituted or substituted heteroaryl.

9. The compound according to claim 8, **characterized in that** R₂ and R₃ are each independently selected from the group consisting of hydrogen, F, Cl and Br;
R₄ is halogen, unsubstituted or substituted C₁-C₃ alkylsulfonyloxy, or unsubstituted or substituted C₆-C₁₂ arylsulfonyloxy, preferably Cl, Br, methanesulfonyloxy or benzenesulfonyloxy; and
R₅ is unsubstituted or substituted C₁-C₃ alkyl, unsubstituted or substituted C₃-C₆ cycloalkyl, unsubstituted or substituted C₁-C₃ alkoxy, unsubstituted or substituted C₆-C₁₂ aryl, or unsubstituted or substituted heteroaryl; preferably, R₅ is selected from the group consisting of methyl, chloromethyl, bromomethyl, dichloromethyl, trichloromethyl, tribromomethyl, methoxy, ethoxy, chlorodifluoromethyl, bromodifluoromethyl, bromodichloromethyl, cyclopropyl, phenyl and substituted phenyl.

10. A compound, represented by formula (VII): 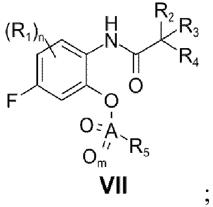 **characterized in that** R₁, R₂, R₃, R₄, R₅, A, m and n are defined as in claim 9.
